**Europäisches Patentamt**

(19) **European Patent Office**

**Office européen des brevets**

(11) Publication number : **0 375 689 B1**

# EUROPEAN PATENT SPECIFICATION

(12)

(45) Date of publication of patent specification :
**12.08.92 Bulletin 92/33**

(51) Int. Cl.⁵ : **A61K 47/00**

(21) Application number : **88905280.9**

(22) Date of filing : **16.05.88**

(86) International application number :
**PCT/US88/01666**

(87) International publication number :
**WO 88/09676 15.12.88 Gazette 88/27**

(54) **A PHARMACEUTICAL COMPOSITION ADAPTED FOR TRANSDERMAL DELIVERY OF AN OPOID DRUG.**

(30) Priority : **01.06.87 US 56520**

(43) Date of publication of application :
**04.07.90 Bulletin 90/27**

(45) Publication of the grant of the patent :
**12.08.92 Bulletin 92/33**

(84) Designated Contracting States :
**AT BE CH DE FR GB IT LI LU NL SE**

(56) References cited :
**EP-A- 0 160 501**
**EP-A- 0 171 742**
**Chemical Abstracts, volume 108, no. 14, 4 April 1988, (Columbus, Ohio, US), T. Loftsson et al.: "The effect of vehicleadditives on the transdermal delivery of nitroglycerin", see page 423, abstract 118806u, & Pharm. Res. 1987, 4(5), 436-7**

(73) Proprietor : **WARNER-LAMBERT COMPANY**
**2800 Plymouth Road**
**Ann Arbor, MI 48105 (US)**

(72) Inventor : **MAHJOUR, Majid**
**82 Koclas Drive**
**Netcong, NJ 07857 (US)**
Inventor : **MAUSER, Bernadette, E.**
**462 Kingsland Avenue**
**Lyndhurst, NJ 07071 (US)**
Inventor : **FAWZI, Mahdi, B.**
**11 Timberline Drive**
**Flanders, NJ 07836 (US)**

(74) Representative : **Mansmann, Ivo**
**c/o Gödecke AG - Patentabteilung Postfach 569 Mooswaldallee 1-9**
**W-7800 Freiburg (DE)**

## Description

This invention relates to pharmaceutical compositions which are useful in effecting transdermal delivery of a therapeutic dose of an opioid drug to the systemic circulation of a mammal.

Many opioids are known to have poor bioavailability in the mammalian systemic circulation due to extensive initial metabolism of the drug by the liver and intestines. Furthermore, the bioavailability of orally administered opioids may be unpredictable since various factors such as changes in acidity and food content can cause changes in the amount of drug absorbed from the gastrointestinal tract. Also, oral administration does not necessarily ensure good patient compliance.

Parenteral administration of opioids provides better bioavailability than oral administration. However, the various routes of parenteral administration such as intravenous, intramuscular, and subcutaneous delivery are not convenient for chronic therapy. This is particularly true for those opioids which exhibit short biological activity half-lives.

Topical formulations of opioids do not necessarily provide delivery of a therapeutic dose of the drug to the systemic circulation and thus provide poor or unpredictable bioavailability. Natural oils containing saturated or unsaturated fatty acids have been described in such topical formulations with drugs used for local anesthetic purposes.

Transdermal delivery of opioid drugs to the mammalian systemic circulation have been described as an alternative mode of administration which can provide the following advantages:

1. Improved and predictable bioavailability of the opioid as compared to oral administration since transdermal delivery avoids initial metabolism by the liver and intestines, and unpredictable absorption from the gastrointestinal tract.

2. A stable blood serum level of the drug resulting in a prolonged pharmacological effect similar to intravenous infusion.

3. Easily adjustable dosing rate which provides maximization of efficacy and minimization of side effects.

4. Easily removable drug source which provides rapid cessation of dosing and elimination of the drug from the body fluids.

5. Convenience of dosing which provides improved patient comfort as compared to parenteral administration and the possibility of greater patient compliance as compared to oral administration.

Transdermal drug delivery is distinguished from topical drug delivery by the fact that while a transdermal formulation is specifically designed to provide a predictable and therapeutically significant rate of delivery of the drug to the systemic circulation, a topical formulation is specifically designed to provide a therapeutic effect only to the local area to which the drug is applied. Furthermore, topical formulations are often designed to prevent any systemic delivery of the drug in order to minimize side-effects. However, even if the topical delivery of a drug does result in systemic absorption, the amount of drug delivery to the circulation is variable and uncontrolled.

European Patent Publication 0 171 742 describes such a system for the transdermal delivery of opioids using saturated or unsaturated fatty alcohols as acids or esters thereof with a carrier or vehicle such as propylene glycol resulting in an organic system, i.e. suspension or gel. The disadvantage of this system is that the use of propylene glycol or other known organic solvents causes irritation to the skin.

It has now been found that saturated or unsaturated fatty acids or esters thereof, such as linoleic acid, is effective as a skin absorption enhancer in purely aqueous systems thus leading to new and effective transdermal compositions without skin irritation.

## SUMMARY OF THE INVENTION

Accordingly the present invention relates to a pharmaceutical composition adapted for transdermal delivery of an opioid drug to the systemic circulation of a mammal containing an opioid drug or a pharmaceutically acceptable salt thereof, characterized in that the composition is an aqueous suspension based on a purely aqueous system containing saturated or unsaturated fatty acids of 8 to 18 carbon atoms or a $C_1$-$C_4$ alkyl ester thereof and a pharmaceutically acceptable excipient to bind the effective ingredients into a cream or lotion form suitable for administration on the skin. Another aspect of the invention is the use of a purely aqueous system containing a saturated or unsaturated fatty acid of 8 to 18 carbon atoms or a $C_1$ to $C_4$ alkyl ester thereof and a pharmaceutically acceptable excipient to bind the effective ingredients into a cream or lotion form for the preparation of an aqueous suspension adapted for transdermal delivery of an opioid drug or a pharmaceutically acceptable salt thereof to the systemic circulation of a mammal.

By the term "opioid" is meant any natural or synthetic opioid analgesic such as morphine, oxymorphone, fentanyl, meperidine, propoxyphene, or oxycodone; any natural or synthetic narcotic antagonist such as nalmefene, naloxone or naltrexone; any natural or synthetic mixed opioid agonist/antagonist such as nalbuphine, butorphanol, buprenorphine or pentazocine; or any pharmaceutically acceptable salt thereof.

By the term "pharmaceutically acceptable salt" is meant any non-toxic pharmaceutically suitable salt of an opioid which has therapeutic properties in mammals. Preparation of such salts is well-known to those skilled in pharmaceuticals. Pharmaceutically acceptable salts of opioids include acetates, naphthylates, tosylates, succinates, hydrochlorides, palmitates, stearates, oleates, pamoates, laurates, valerates, hydrobromides, sulfates, methane sulfonates, tartrates, citrates, and maleates.

The term "saturated or unsaturated fatty acid of 8-18 carbon atoms" means any such acid or ester thereof effective in enhancing the penetration of a drug through the mammalian skin. Preferred are linoleic and oleic acids and their $C_1$-$C_4$ alkyl esters. Most preferred is linoleic acid.

Pharmaceutically acceptable excipients are additional materials used in the compositions to bind the effective ingredients into a cream or lotion form suitable for administration on the skin per se or through known devices such as bandaids, tapes and patches. These excipients are, for example, carbopol 934, carbopol 940, carbopol 941, (B. F. Goodrich and Co. they are acrylic acid, water soluble resin polymers, with molecular weights of 3,000,000; 4,000,000; and 1,250,000 respectively); tween 20, (ICI Americas) polysorbate 20 polyoxyethylene 20 sorbitan monolaurate, or other tweens such as tween 40, tween 60, and tween 80, and other pharmaceutically acceptable emulsifiers such as polyethyleneglycol esters, e.g. polyethyleneglycol monolaurates, can also be used.

The effectiveness of the present invention is illustrated by the following examples and results illustrated in table form which compares the permeation of oxymorphone through hairless mouse skin from organic and aqueous enhancer systems containing linoleic acid.

· Examples

Non Aqueous Systems

| Formulation | Flux ($\mu$g/cm$^2$/h) | P (cm/sec x 10$^6$) | Lag Time (h) | Maximum Solubility (mg/ml) | Maximum* Flux (PxSol$_Y$) |
|---|---|---|---|---|---|
| LA:PG:TA 20:30:50 | 66.6 | 3.49 | 3.5 | 130.17 | 1635.46 |
| LA:PG:TA 10:30:60 | 51.5 | 2.65 | 4.2 | 93.77 | 894.29 |
| LA:PG:TA 5:30:65 | 40.0 | 2.02 | 7.6 | 60.60 | 440.68 |
| PG:TA 37.5:62.5 | < 3 | -- | -- | -- | -- |

*Calculated based on Fick's Law (flux = permeability x concentration gradient; maximum flux = P x solubility of drug in donor solution), assuming Fick's Law holds.

EP 0 375 689 B1

## Aqueous Systems

| Formulations (containing 5% w/w oxymorphone | Flux ($\mu g/cm^2/h$) | Lag Time (h) |
|---|---|---|
| LA 30% (0.3% Carbopol + 2.5% Tween 20) 70% | 667.45 | 6.8 |
| LA 20% (0.3% Carbopol + 2.5% Tween 20) 80% | 636.11 | 9.3 |
| LA 10% 0.3% Carbopol + 2.5% Tween 20) 90% | 672.76 | 4.3 |
| LA 5% (0.3% Carbopol + 2.5% Tween 20) 95% | 543.82 | 9.5 |
| LA 20% (2.5% Tween 20) 80% | 884.46 | 4.4 |
| 0.3% Carbopol | 38.31 | 17 |
| 0.3% Carbopol + 2.5% Tween 20 | 19.73 | 15.61 |

Legend

LA = Linoleic Acid

PG = Propylene Glycol

TA = Triacetin

Note: Since the aqueous systems are suspensions, they are constantly providing maximum availability of oxymorphone or permeation (i.e. maximum flux); therefore, to compare permeability data with the organic systems, maximum flux values had to be calculated. Using the premeability coefficients for 0.5% oxymorphone solutions in the linoleic acid:propylene glycol:triacetin mixtures, maximum fluxes were calculated by multiplying the saturation solubility of oxymorphone in the respective system by its corresponding permeability coefficient. However, it should be noted that the aqueous dispersions (5% w/w drug) became depleted of drug causing a plateau in cumulative average concentration versus time graphs, therefore, higher flux values may be anticipated with the aqueous systems.

As shown in the table, aqueous systems containing the model fatty acid, linoleic acid, effectively enhanced the permeation of a model drug through the skin. The usual dose of oxymorphone is 6-10 mg per day which would be adequately provided by any of the aqueous systems containing linoleic acid from a 10 cm² patch.

## Claims

1. A pharmaceutical composition adapted for transdermal delivery of an opioid drug to the systemic circulation of a mammal, containing an opioid drug or a pharmaceutically acceptable salt thereof, characterized in that the composition is an aqueous suspension based on a purely aqueous system containing saturated or unsaturated fatty acids of 8 to 18 carbon atoms or a $C_1$-$C_4$ alkyl ester thereof and a pharmaceutically acceptable excipient to bind the effective ingredients into a cream or lotion form suitable for administration on the skin.

2. A composition according to Claim 1, wherein the opioid is a natural or synthetic opioid analgesic such as morphine, oxymorphone, fentanyl, meperidine, propoxyphen, or oxycodone; a natural or synthetic narcotic antagonist such as nalmefene, naloxone, or naltrexone; a natural or synthetic mixed opioid agonist/antagonist such as nalbuphine, butorphanol, buprenorphine or pentazocine; or a pharmaceutically acceptable salt thereof.

3. A composition according to Claim 2, wherein the opioid is oxymorphone.

4. A composition according to Claim 1, wherein the fatty acid is linoleic or oleic acid.

5. A composition according to Claim 1, wherein the aqueous suspension contains up to 0.1-10% by weight of drug.

6. A composition according to Claim 1, wherein the aqueous suspension contains from 1 to 30% by weight of a saturated or unsaturated fatty acid of 8-18 carbon atoms or a $C_1$-$C_4$ alkyl ester thereof.

7. A composition according to Claim 6, wherein the aqueous suspension contains from 1 to 20% by weight of linoleic or oleic acid or a $C_1$-$C_4$ alkyl ester thereof.

8. A composition according to Claim 6, wherein the aqueous system contains about 1 to 30% by weight of linoleic acid.

9. A composition according to Claim 6, wherein the aqueous system contains 10 to 20% by weight of linoleic acid.

10. Use of a purely aqueous system containing a saturated or unsaturated fatty acid of 8 to 18 carbon atoms or a $C_1$ to $C_4$ alkyl ester thereof and a pharmaceutically acceptable excipient to bind the effective ingredients into a cream or lotion form for the preparation of an aqueous suspension adapted for transdermal delivery of an opioid drug or a pharmaceutically acceptable salt thereof to the systemic circulation of a mammal.

**Patentansprüche**

1. Pharmazeutische Zusammensetzung für die transdermale Freisetzung eines Opioids in die systemische Zirkulation eines Säugers, enthaltend ein Opioid oder ein pharmazeutisch verträgliches Salz davon, dadurch gekennzeichnet, daß die Zusammensetzung, eine wäßrige Suspension, welche auf einem reinen, wäßrigen System, enthaltend gesättigte oder ungesättigte Fettsäuren von 8 bis 18 Kohlenstoffatomen oder eines $C_1$-$C_4$ Alkylesters davon, basiert, und einen pharmazeutisch verträglichen Exzipienten, um die wirksamen Bestandteile in eine für die Verabreichung auf der Haut geeignete Creme oder Lotion einzubinden, enthält.

2. Zusammensetzung nach Anspruch 1, worin das Opioid ein natürliches oder synthetisches, analgetisches Opioid, wie Morphin, Oxymorphon, Fentanyl, Meperidin, Propoxyphen oder Oxycodon; ein natürlicher oder synthetischer Narkotikaantagonist, wie Nalmefen, Naloxon oder Naltrexon; ein natürlicher oder synthetischer, gemischter Opioid Agonist/Antagonist, wie Nalbuphin, Butorphanol, Buprenorphin oder Pentazocin, oder ein pharmazeutisch verträgliches Salz davon ist.

3. Zusammensetzung nach Anspruch 2, worin das Opioid Oxymorphon ist.

4. Zusammensetzung nach Anspruch 1, worin die Fettsäure Linolsäure oder Ölsäure ist.

5. Zusammensetzung nach Anspruch 1, worin die wäßrige Suspension 0,1 bis 10 Gew.-% Wirkstoff enthält.

6. Zusammensetzung nach Anspruch 1, worin die wäßrige Suspension 1 bis 30 Gew.-% einer gesättigten oder ungesättigten Fettsäure von 8 bis 18 Kohlenstoffatomen oder eines $C_1$-$C_4$ Alkylesters davon enthält.

7. Zusammensetzung nach Anspruch 6, worin die wäßrige Suspension 1 bis 20 Gew.-% Linolsäure oder Ölsäure oder eines $C_1$-$C_4$ Alkylesters davon enthält.

8. Zusammensetzung nach Anspruch 6, worin das wäßrige System etwa 1 bis 30 Gew.-% Linolsäure enthält.

9. Zusammensetzung nach Anspruch 6, worin das wäßrige System 10 bis 20 Gew.-% Linolsäure enthält.

10. Verwendung eines rein wäßrigen Systems, enthaltend eine gesättigte oder ungesättigte Fettsäure von 8 bis 18 Kohlenstoffatomen oder einen $C_1$-$C_4$ Alkylester davon und einen pharmazeutisch verträglichen Exzipienten zum Einbinden der wirksamen Bestandteile in Form einer Creme oder Lotion zur Herstellung einer wäßrigen Suspension, welche für die transdermale Freisetzung eines Opioids oder eines pharmazeutisch verträglichen Salzes an die systemische Zirkulation eines Säugers adaptiert ist.

**Revendications**

1. Une composition pharmaceutique adaptée à l'administration transdermique d'un médicament opiacé dans la circulation systémique d'un mammifère, contenant un médicament opiacé ou un de ses sels pharmaceutiquement acceptables, caractérisée en ce que la composition est une suspension aqueuse basée sur un système purement aqueux contenant des acides gras saturés ou insaturés renfermant de 8 à 18 atomes de carbone ou un de leurs alkylesters en $C_1$-$C_4$ et un excipient pharmaceutiquement acceptable pour lier les ingrédients efficaces sous forme de crème ou de lotion convenant à une administration sur la peau.

2. Une composition selon la revendication 1, caractérisée en ce que l'opiacé est un analgésique opiacé naturel ou synthétique, notamment morphine, oxymorphone, fentanyl, mépéridine, propoxyphène ou oxycodone; un antagoniste narcotique naturel ou synthétique notamment nalméfène, naloxone ou naltrexone; un agoniste/antagoniste opiacé mixte naturel ou synthétique, notamment nalbuphine, butorphanol, buprénorphine ou pentazocine; ou un de leurs sels pharmaceutiquement acceptables.

3. Une composition selon la revendication 2, caractérisée en ce que l'opiacé est l'oxymorphone.

4. Une composition selon la revendication 1, caractérisée en ce que l'acide gras est l'acide linoléique ou l'acide oléique.

5. Une composition selon la revendication 1, caractérisée en ce que la suspension aqueuse contient de 0,1 à 10% en poids de médicament.

6. Une composition selon la revendication 1, caractérisée en ce que la suspension aqueuse contient de 1 à 30% en poids d'un acide gras saturé ou insaturé renfermant de 8 à 18 atomes de carbone ou un de ses alkylesters en $C_1$-$C_4$.

7. Une composition selon la revendication 6, caractérisée en ce que la suspension aqueuse contient de 1 à 20% en poids d'acide linoléique ou d'acide oléique ou l'un de leurs alkylesters en $C_1$-$C_4$.

8. Une composition selon la revendication 6, caractérisée en ce que le système aqueux contient environ 1 à 30% en poids d'acide linoléique.

9. Une composition selon la revendication 6, caractérisée en ce que le système aqueux contient 10 à 20% en poids d'acide linoléique.

10. Utilisation d'un système purement aqueux contenant un acide gras saturé ou insaturé renfermant de 8 à 18 atomes de carbone ou un de ses alkylesters en $C_1$ à $C_4$ et un excipient pharmaceutiquement acceptable pour lier les ingrédients efficaces sous forme de crème ou de lotion pour la préparation d'une suspension aqueuse adaptée à l'administration transdermique d'un médicament opiacé ou de l'un de ses sels pharmaceutiquement acceptables dans la circulation systémique d'un mammifère.